# EUROPEAN PATENT SPECIFICATION

(11) **EP 2 211 898 B1**
(45) Date of publication and mention of the grant of the patent: **07.12.2016**
(21) Application number: 08838897.0
(22) Date of filing: 20.10.2008
(51) Int. Cl.: A61K 38/48, A61K 38/49, A61P 9/10

(54) **NOVEL PATIENT SUBGROUPS FOR THROMBOLYSIS**
NEUE PATIENTENSUBGRUPPE FÜR APOPLEXIE-PATIENTEN
TRAITEMENT AMÉLIORÉ DE PATIENTS AYANT SUBI UN ACCIDENT VASCULAIRE CÉRÉBRAL

(30) Priority: 18.10.2007 EP 07020401; 26.11.2007 EP 07022867; 15.10.2008 EP 08017954
(43) Date of publication of application: 04.08.2010
(73) Proprietor: H. Lundbeck A/S, 2500 Valby (DK)
(72) Inventor: SÖHNGEN, Mariola, 52080 Aachen (DE); EBEL, Alice, 52078 Aachen (DE); AL-RAWI, Yasir, Alaa, Shafeek, Sharjah (AE)
(74) Representative: von Renesse, Dorothea
(86) International application number: PCT/EP2008/008871
(87) International publication number: WO 2009/049914

(56) References cited:
- WO-A-2005/018564
- US-A1- 2006 135 425
- MAULAZ A, PIECHOWSKI-JOWIAK B, MICHEL P, BOGOUSSLAVSKY J: "Selecting patients for early stroke treatment with penumbra images" CEREBROVASC DIS, vol. 20(suppl2), 2005, pages 19-24, XP009094739

## Description

Stroke is the third leading cause of death, after cardiovascular disease and cancer. Each year, stroke is diagnosed in 750,000 patients and contributes to nearly 168,000 deaths in the United States only. Stroke has a high personal and social impact because of the severe disability that the disease causes.

The administration of thrombolytics - such as plasminogen activators - to the patient in acute ischemic stroke therapy aims to rescue the "tissue at risk" (which is sometimes in the scientific literature also referred to as "penumbra") and to reduce the final infarct size, thereby improving patient clinical outcome. Presently available and approved regimes for intravenous thrombolytic therapy are based on the time interval after the onset of the stroke symptoms. In other words, only patients with a stroke onset no later than 180 min (3 hours) before treatment are considered to be treatable with an approved thrombolytic treatment. Recent clinical studies ("ECASS-3") with the rt-PA alteplase established that rt-PA is effective even 3 to 4.5 hours after stroke onset. However in that extended time window a significantly increase incidence of intracranial hemorrhage (ICH) was observed (Hacke W. et al.: "Thrombolysis with Alteplase 3 to 4.5 Hours after Acute Ischemic Stroke", in: The New England Journal of Medicine 2008 Vol. 359, No 13, p. 1317-1329). Thus severe safety concerns remain when extending the time window for stroke treatment with alteplase.

Before the treatment, patients for thrombolytic therapy undergo non-contrast cerebral computed tomography (CT) evaluations in order to confirm the absence of cerebral hemorrhage and the absence of a cerebral hypodensity encompassing more than one third of the middle cerebral artery (MCA) territory. They are also evaluated to exclude contraindications for thrombolysis constituting risk factors for hemorrhage (e.g. a malignant tumors, recent trauma, recent surgery, a brain tumor, a vascular malformation or an aneurysm).

However, even with such restrictive criteria, thrombolysis is associated with a significant risk of intracranial hemorrhage (ICH), which occurs up to 15% of all treated patients. The clinical benefit of the currently available thrombolytic therapy does not compensate for this risk. Thus, selection of patients to be included in a thrombolysis protocol according to the current criteria is not sufficiently satisfactory.

Accordingly, it is the objective of the present invention to suggest novel patient subgroups which will benefit from stroke treatment and therewith exclude those patients who are not assumed to profit from thrombolysis. Therewith the overall efficacy and safety of thrombolytic stroke therapy should be increased.

It has now been found by the inventors that the selection of patients for thrombolytic therapy only on the basis of individual imaging of stroke patients and time lapse since stroke onset bears the risk of including patients with only minor or transient stroke events into the treatment, and therewith expose these patients to the risk of ICH without an underlying medical need. Furthermore it has been found, that the number of patients eligible for stroke treatment can reasonably be limited to those patients, who exhibit a stroke event, which cannot be overcome by self-healing capacities and thus need medical treatment. These stroke events can be categorized as being educible by certain properties as outlined below. Accordingly in one embodiment of the invention distinct subgroups of patients are selected for the treatment with thrombolytics.

According to one embodiment, the selected patient subgroups will undergo a
process of individual imaging before treatment for assessing the possible tissue at risk which indicates potentially salvageable brain tissue. In another embodiment of the invention the patients are selected as to exhibit an artery occlusion. And yet a further embodiment of the invention the patients are selected as to tissue at risk and artery occlusion.

According to further embodiments, the selected patients can be treated with a
bolus injection of the non-neurotoxic plasminogen activator desmoteplase. In one embodiment of the invention a bolus of either about 90 or about 125 microgram plasminogen activator per kg body weight can be administered. The treatment can be initiated later than three hours after stroke onset.

### Background of the invention

The target tissue of thrombolytic therapy is the so called tissue at risk. The pathopysiological rationale behind this is as follows:
Immediately after occlusion of an artery supplying the brain, the regional cerebral blood flow (rCBF) decreases. Brain tissue with critically low perfusion, referred to as "ischemic tissue", first loses function and finally integrity due to a lack of glucose and oxygen. The area where the integrity of brain tissue is largely lost is known as the "infarct core" and develops within the first minutes of vessel occlusion at the center of the ischemic area. This infarct core is characterized by irreversible neuronal cell damage and is surrounded by ischemic, but still salvageable tissue at risk of infarction. The tissue at risk is also referred to as the "penumbra".

Depending on the time lapse since stroke onset and the severity of cerebral ischemia, the quantity of collateral flow, and the metabolic status of the patient, the tissue at risk eventually loses its structural integrity and thus progress to infarction.

In the case of persistent vessel occlusion, the core of infarction expands over time until nearly all of the tissue at risk has progressed to infarction. However, in the case of early recanalization of the feeding vessel, the ischemic changes within the tissue at risk are reversible, and it can potentially be salvaged. Therefore, the tissue at risk is the target of current thrombolytic therapy.

Although the above-mentioned factors for the development of infarction as such are known, there is a lack of understanding of possible interactions and consequences. Accordingly, a convincing concept for the selection of treatable patient groups acceptable for the clinical routine use is still missing. In order to minimize the risk of hemorrhagic transformation, the only thrombolytic treatment presently approved is strictly limited to a maximum of 3 hours time from the onset of stroke symptoms.

### Summary of the invention

In contrast to the present thrombolytic therapy, the invention is based on the one hand on the individual assessment (diagnosis) of the tissue at risk (penumbra) of the patient, regardless of the time lapse after stroke onset. On the other hand the invention is based on the selection (diagnosis) of stroke patients suffering from a stroke which is due to an occlusion within a cerebral blood vessel. In one embodiment the occlusion is detectable by means of an imaging tool. Accordingly the patients for stroke treatment are selected in view of their tissue at risk and/or for artery vessel occlusion.

As used for the purpose of the invention the term "occlusion" is defined as any stricture or narrowing of a blood vessel which results in a reduced blood flow of the tissue distal thereof compared to the healthy or normal blood vessel. The occlusion can either be partial or complete. Hence, the term occlusion encompasses also a stenosis, i.e. an abnormal narrowing of a blood vessel still allowing distal perfusion.

According to the disclosure any imaging tool can be applied which results in the visualization of the inner opening of structures filled with blood and therewith enables the identification of an arterial occlusion. Possible imaging modalities include MR angiography (MRA) or CT angiography (CTA) and further developments or modifications thereof; however without being limited to it. The visualization of blood vessels can also be referred to as angiography. Various methods for the further evaluation of MRI or CT images are known to the person skilled in the art (e.g. MTT, TTP or Tmax as post processing maps).

In a further embodiment, the occlusion is localized in a proximal cerebral artery, in particular the middle cerebral artery (MCA), the anterior cerebral artery (ACA) and/or the posterior cerebral artery (PCA) including all of their branches, in particular M1 and/or M2.

The patient subgroup selected for thrombolysis has an occlusion at baseline which is describable by a TIMI grade of 0 or 1. The grade 0 means complete occlusion.

A TIMI grade of 1 or less (i.e. 0 or 1) is referred to as a "high grade stenosis". The occlusion in the M1 and/or M2 of the proximal cerebral artery is preferably of a TIMI grade 0 or 1.

The TIMI scale (Thrombolysis in Myocardial Infarction scale) was originally developed for the assessment of arterial occlusions in myocardial infarction and encompasses 4 grades as follows:
grade 3: normal blood flow
grade 2: artery entirely perfused but blood flow delayed
grade 1: artery penetrated by contrast material but no distal perfusion
grade 0: complete occlusion of the vessel.

The TIMI scale was established in a myocard infarction trial and is since then known to the person skilled in thrombolysis, e.g. from Chesebro JH et al: "Thrombolysis in Myocardial Infarction (TIMI) Trial, Phase I: A comparison between intravenous tissue plasminogen activator and intravenous streptokinase. Clinical findings through hospital discharge", in: Circulation 1987; 76; 142-154.

The selected patient group suffers a stroke which can be described by an NIHSS score of at least 4, preferably up to and including 24. However, the invention can also pertain to stroke patients with a NIHSS score of at least 8.

Hence, the group of patients selected for stroke treatment is characterized by a TIMI grade of 1 or 0 (i.e. a TIMI grade of less than 2), in particular in an M1 and/or M2 proximal cerebral artery, and an NIHSS score of at least 4. In a particular embodiment the NIHSS score is at least 8 to 24 (inclusive). The patients preferably show clinical signs of hemispheric infarction. The group of patients selected for stroke treatment is additionally characterized by cerebral tissue at risk and a cerebral artery occlusion.

Hence, turning away from the fixed 3 hour time window of present therapies, according to one embodiment of the invention, patients *without* tissue at risk are not considered to be treatable, even if they present at the hospital within the 3 hour time-window currently approved for rt-PA; whereas patients *with* tissue at risk and artery vessel occlusion are open to receive thrombolytic medication, even if they arrive at the hospital *later* than the 3 hours after stroke onset.

Imaging techniques can be used for the assessment of tissue at risk and/or vessel occlusion, since they make it possible to obtain deeper insights into pathophysiological parameters in ischemic stroke. Imaging techniques can identify patients with an ischemia exceeding the infarct core, and who thus constitute the target population of the thrombolysis therapy according to this embodiment of the invention. Thus, pathophysiologically-based imaging in order to assess the tissue at risk as well as the infarcted core is a way to determine whether a patient is susceptible to the treatment according to the invention. Any imaging technique capable of assessing the tissue at risk and/or vessel occlusion is suitable, such as e.g. MRI or CT; however without being limited to it.

Imaging can also be used to ensure patient safety by excluding individuals at high risk for post-treatment hemorrhage and those with low likelihood of benefit because there is no demonstrable tissue at risk. Risk factors, which on a regular basis may exclude the treatment according to the invention (though not always), are the evidence of intracranial hemorrhage (ICH), subarachnoid hemorrhage (SAH), arteriovenous malformation (AV), cerebral aneurysm or cerebral neoplasm. Furthermore, according to one embodiment of the invention, patients with an acute infarction involving more than approximately 1/3 of the territory of the middle cerebral artery (MCA) or substantially the entire territory of the anterior cerebral artery (ACA) and/or the posterior cerebral artery (PCA) can be excluded from treatment by the invention. In addition, patients with signs of Blood Brain Barrier (BBB) leakage represent a risk factor for thrombolytic therapy and - according to one embodiment of the invention - should be excluded.

Although the concept of individual imaging is not limited to a certain time window, it can be favorable to treat the patients within a time window of up to 9 hours from stroke onset; i.e. the treatment is possible even later than 3 hours after stroke onset.

As mentioned above the patient subgroups selected according to the invention suffer from a stroke which needs medical treatment. These subgroups are characterized by one or more clinical properties as outlined in detail below.

The efficacy of the stroke treatment according to the invention can be shown by assessing the difference of percentage change of the core lesion volume from pre-treatment imaging assessment to day 30 after treatment between the groups with active treatment (verum) and placebo or with the comparison of the clinical response rate at day 90.

In one embodiment, the treatment of the selected stroke patients can comprise the administration of about 50 to 125 microgram of the plasminogen activator desmoteplase per kg body weight of the patient, in particular from about 90 to about 125, in particular 90 or 125 microgram per kg body weight of the patient. In a preferred embodiment 90 or 125 microgram per kg body weight of DSPA alpha 1 is administered.

In a further embodiment, patients are excluded who do not suffer an M1 or M2 MCA occlusion and/or a mismatch volume of below about 120, 100 cc, in particular 75 cc or 50 cc or less at baseline. Hence the patients for treatment can be selected for exhibiting an absolute mismatch volume of at least about 50, 75 or 100 or 120 cc at baseline.

### Detailed description of the invention

As outlined above, individual imaging is used to diagnose or
identify (select) candidates for the thrombolytic (recanalization and/or reperfusion) therapy. Any suitable imaging tool can be used. For example, MRI is an imaging tool that can be applied, which can be performed with a diffusion-weighted sequence (DWI). Strong hyperintensity on DWI indicates a core lesion destined to infarction with or without therapeutic reperfusion. It is normally surrounded by a hypoperfused region measured with PWI (Perfusion Weight Imaging).

Some patients show a hyperintensity on DWI which covers nearly the entire volume of hypoperfused tissue identified by PWI. This "match" of lesion size on DWI and PWI indicates minimal tissue at risk. Patients selected according to one embodiment of the invention present a PWI lesion distinctively larger than the DWI lesion and thus present a "mismatch", that indicates a potentially salvageable region of tissue at risk (penumbra). The region of the tissue at risk favorably is by at least about 20% larger than the region of the core infarct. The tissue at risk can be located e.g. in the area of the middle cerebral artery (MCA), the area of anterior cerebral artery (ACA) or the area of posterior cerebral artery (PCA). In an embodiment of the invention, these groups of "mismatch" patients or patients with a penumbra, which is at least about 20% larger than the core infarct, are subject of the thrombolytic therapy.

Furthermore, MRA (Magnetic Resonance Angiography) can be used in order to identify the site of vessel occlusion. In cases where vessel occlusion persists, the formerly salvageable tissue at risk will likely infarct. After early recanalization perfusion of the tissue at risk will be normalized leading to tissue salvage.

MR as an imaging tool is named as an example only. The penumbra ("tissue at risk") identification is also possible e.g. with CT using the perfusion CT (PCT) method, or Positron Emission tomography (PET). Another example is ultrasound visualization.

The NIHSS is a systematic assessment tool that provides a quantitative measure of stroke-related neurologic deficit. The NIHSS was originally designed as a research tool to measure baseline data on patients in acute stroke clinical trials. Now, the scale is also widely used as a clinical assessment tool to evaluate acuity of stroke patients, determine appropriate treatment, and predict patient outcome. According to the NIHSS, parameters such as the level of consciousness, the eye movement, the facial palsy or the motor ability of arms or legs are assessed and subject to a pre-defined numerical scoring. On a regular basis a NIHSS score of 6 or below is considered as a rather light stroke, whereas a NIHSS score from 6 to approximately 15 is qualified as a stroke of medium severity. A score of 15 or more of the NIHSS scale indicates a rather severe stroke. Frequently a stroke of an NIHSS score of 20 or more is considered as being untreatable. However, notably the qualification of the severity of a stroke depends also on the individual assessment of the patient by the physician, which includes aspects of the overall clinical performance of the patient. According to one embodiment of the invention a baseline NHISS score of at least 4 or of at least 8 is required. The maximum score can be selected to be 24. Hence on one embodiment to NIHSS score at baseline is from 4 to 24 (inclusive) or 8 to 24 (inclusive).

As outlined above, imaging can be applied also the exclude certain patient groups from thrombolysis, namely in order to exclude certain risk factors. Accordingly in one embodiment , the selected patient groups do not exhibit one or more of the following properties
o acute infarction involving more than around 1/3 of MCA or substantially the entire ACA and/or PCA territory
o evidence of ICH, SAH, AV malformation, cerebral aneurysm or cerebral neoplasm.

These patients are favorably treated later than 3 hours, later than 4,5 hours or later than 6 hours after stroke onset. Most preferred they are treated within 3 to 9 hours after the onset of stroke symptoms.

The clinical outcome can e.g. be measured as a "clinical response rate" at day 90 after treatment, which e.g. is defined as having achieved one or more of the three parameters as follows:
i. at least 8 point NIHSS improvement or a final NIHSS score of 0-1 at day 90, e.g. an improvement from NIHSS score from 24 to 16 or to 0-1 (if the patient at baseline had a NIHSS score of 9 or below)
ii. a score in the modified Rankin Scale (mRS) of 0-2
iii.a Barthel Index between approximately 75 to approximately 100.

Furthermore, these groups of patients can show a reduction of the infarct core lesion volume at day 30 compared to the pre-treatment state (baseline).

The plasminogen activator used for the stroke treatment can be administered to the patient as a single bolus injection with the plasminogen activator desmoteplase, at a dose of about 50 to 125 micrograms per kg body weight, in particular with about 90 or about 125 micrograms per kg body weight of the patients. Hence the invention also pertains to the manufacture of a medicament (i.e. a certain dosage unit form) for treating selected stroke patients comprising a dosage, which allows the preparation of a ready-to-use formulation comprising of about 50 to 125 micrograms per kg body weight, or about 90 or about 125 micrograms/kg body weight. The dosage unit form can be, e.g. a solid such as a lyophilisate, or a liquid in a vial or ampul. In an embodiment the dosage unit form contains about 5.0 to 12.5 mg, preferably about 9.0 or about 12.5 mg of the non-neurotoxic plasminogen activator desmoteplase.

The patients selected show a reduction of the infarct core lesion
volume at day 30 compared to the pre-treatment state (baseline).

The present disclosure also relates to a thrombolytic treatment which can be performed with any plasminogen activator (PA). As used herein the term "plasminogen activator" refers to all substances - either naturally or synthetically provided, with human origin or non-human origin - which stimulate via the proteolytic activation of plasminogen to plasmin the clot lysis. Typical PAs known to the skilled person are, e.g., the tissue plasminogen activator (PA), which is available in its recombinant form rtPA (alteplase), streptokinase or urokinase, and their respective derivates, fragments or mutants, which maintain the proteolytic activity (e.g. tenecteplase or reteplase for rtPA).

In one embodiment of the disclosure, a non-neurotoxic plasminogen activator is used, i.e., a plasminogen activator, which *per se* exhibits a substantially reduced potential to activate the NMDA type glutamate receptor. This plasminogen activator favorably is essentially non-activatable by beta-amyloid or prion protein and shows in the presence of fibrin an enhanced activity of more than about 550 fold, more than about 5500 fold, or more than about 10,000 fold, compared to the activity in the absence of fibrin. In yet an embodiment, the increase of activity of the PA in the presence of fibrin compared to its activity in the absence of fibrin is more than about 100,000. Since the increase in activity of rt-PA is about 550, in one embodiment of the disclosure a PA is used which has an approximately 180-200 fold higher fibrin specificity/selectivity compared to rt-PA.

The neurotoxicity can be assessed by methods known to the skilled person, e.g. with animal models in particular kainic acid models as described in detail in the international laid open WO03/037363. This model is further described in detail in Liberatore et al. (Liberatore, G.T.; Samson, A.; Bladin, C.; Schleuning, W.D.; Medcalf, R.L. "Vampire Bat Salivary Plasminogen Activator (Desmoteplase)", Stroke, February 2003, 537-543) and Reddrop et al. (Reddrop, C.; Moldrich, R.X.; Beart, P.M.; Liberatore, G.T.; Howells, D.W.; Schleuning, W.D.; Medcalf, R.L., "NMDA-mediated neurotoxicity is potentiated by intravenous tissue-type-, but not vampire bat-plasminogen activator, and is enhanced by fibrin", Monash University Department of Medicine, Version November 20, 2003).

In yet another embodiment of the disclosure the PA has a plasma half-life of more than 2.5 min, more than 50 min, or more than 100 min.

In an embodiment, DSPA alpha 1 or PA with a biological activity and pharmacological properties essentially corresponding to DSPA alpha 1 regarding the activation of plasminogen and its enhanced fibrin selectivity/specificity are used. DSPA alpha 1 has a half-life of about 138 min and a 105,000 fold increased activity in the presence of fibrin compared to its activity in the absence of fibrin.

DSPA alpha 1 is a plasminogen activator, which originally was isolated or derived from the saliva of *Desmodus rotundus (**D**esmodus* **S**alivary **P**lasminogen **A**ctivator). Within the saliva, four variants of DSPA had been isolated which, similarly to alteplase and urokinase, are composed of various conserved domains previously established in related families of proteins. The variants rDSPA alpha1 and rDSPA alpha2 exhibit the structural formula Finger (F), Epidermal Growth Factor (EGF) (sometimes also referred to as "(E)"), Kringle (K), Protease (P), whereas rDSPA beta and rDSPA gamma are characterized by the formulas EKP and KP, respectively. Subtle sequence differences and data from southern blot hybridisation analysis indicate that the four enzymes are coded by four different genes and are not generated by differential splicing of a single primary transcript.

The variant DSPA alpha 1 has an at least 70% structural homology to alteplase (rt-PA); the difference being that alteplase has two kringles (FEKKP), whereas DSPA alpha 1 has only one (FEKP). DSPA alpha 1 is a serine protease with 441 amino acids. Like other plasminogen activators (PA), DSPA alpha 1 activates plasminogen by catalysing the conversion of plasminogen into plasmin, which in turn breaks down the cross-linked fibrin abundant in blood clots.

DSPA alpha 1 has been found to have a high specificity for plasminogen-bound fibrin, high fibrin selectivity (defined by activation by fibrin relative to activation by fibrinogen), substantially no neurotoxicity, and negligible activation by beta-amyloid and human cellular prion protein, in addition to a long dominant half-life of more than 2 hours (see above).

Recombinant DSPA alpha 1 can be obtained from Chinese hamster ovary cells containing a recombinant plasmid carrying the DSPA alpha 1 gene from *Desmodus rotundus.* **Fig. 1** and **fig. 2** show the structures of DSPA alpha 1 and alteplase. The sequence of the mature DSPA alpha 1 is shown in **fig. 3****.**

The plasminogen activators from *Desmodus rotundus* and their recombinant forms were first disclosed in US patent Nos. 6,008,019 and 5,830,849. US 6,008,019 discloses the sequence data of DSPA alpha 1. Both patents relate to the structure, properties and manufacture of plasminogen activators from *Desmodus rotundus,* in particular DSPA alpha 1. The recombinant manufacture and further processing is also subject of the EP 1 015 568 B1, disclosing the recombinant manufacture of DSPA alpha 1.

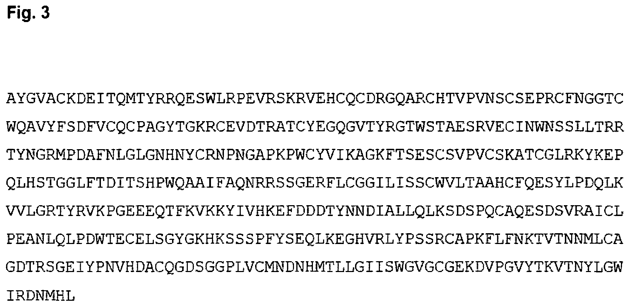

According to the present invention, the term "desmoteplase" is used for any plasminogen activator with identical or essentially the same biological activity of DSPA alpha 1 regarding the activation of plasminogen and its enhanced fibrin selectivity/specificity. In a further embodiment the fibrin selectivity is at least 180 fold compared to rt-PA. The PAs defined as desmoteplase according to the invention can be at least 80 or 90%, at least 95 %, or at least 98% identical to the amino acid sequence according to fig. 3 (DSPA alpha 1). The plasminogen activators can include microheterogeneities, e.g. in terms of glycosylation and/or N-terminal variations, which are merely due to production systems.

### EXAMPLE 1

Clinical Benefit of Desmoteplase Treatment in Patients with Moderate to Severe Stroke: Results of the DIAS-2 Trial

**BACKGROUND:** Desmoteplase in Acute Ischemic Stroke-2 (DIAS-2) was a randomized, placebo-controlled, double-blind study that investigated the safety and efficacy of Desmoteplase, DSPA, (90 and 125 mcgm/kg) in acute stroke within 3-9 hours after onset of symptoms. The negative intent-to-treat analysis results and an atypically high placebo response rate (46%) prompted a more detailed analysis of clinical and imaging data.

**METHODS:** Patients age 18-85, NIHSS 4-24, with a visually apparent penumbral mismatch pattern by investigator judgment on CT (n= 64) or MRI (n=122) were randomized to placebo (n=63), 90 mcgm/kg (n=57) or 125 mcgm/kg (n=66) of DSPA. Images were centrally processed and blindly assessed. Clinical response, the primary outcome of the trial, was an improvement on all three stroke scales (NIHSS, mRS and Barthel Index) at 90 days. CT-selected patients were not included in mismatch-based analyses because volume measurements and their clinical correlations were more variable.

**RESULTS:** DIAS-2 patients had less severe strokes than in previous DSPA trials (DEDAS and DIAS): median NIHSS=9 vs 12; 46% (82/179) vs 37% (33/89) had no M1/M2 MCA occlusion. In MRI patients, median baseline core lesion volume was 9.7 cc and median mismatch volume was 78.4 cc. As absolute mismatch volume at baseline increased, the placebo response rate declined relative to DSPA. Highest placebo response rates were found in patients with absolute mismatch volume < 75 cc (67%; 12/18) or absence of M1 or M2 MCA occlusion (63%; 12/19). Excluding these mild patients clinical response rates were 27% for placebo (6/22) and 46% for DSPA (25/54) in the MRI population. Clinical outcomes reported for DIAS and DEDAS were 23% for placebo and 49% for 90 or 125 mcgm/kg DSPA. Pooling all MR-selected patients in the 3 trials, clinical response rates were 34% for placebo (n=73) and 48% for 90 or 125 mcgm/kg DSPA (n=141).

**CONCLUSIONS:** The mild strokes included in DIAS-2 may explain the unexpectedly high placebo response rate. A proven M1/M2 MCA occlusion or an MRI-mismatch volume >75 cc at baseline was associated with better clinical outcomes for DSPA in DIAS-2, supporting the positive efficacy results achieved in DIAS and DEDAS. A further trial with DSPA using refined imaging and clinical selection criteria is being planned.

### EXAMPLE 2

Clinical Benefit of Desmoteplase Treatment in Patients with Moderate to Severe Stroke: Further results of the DIAS-2 Trial based on an in-depth re-analysis of the DIAS-2 data.

### BACKGROUND: see above

**METHODS:** see above

### RESULTS:

### Responder rates vs. TIMI grade

The DIAS/DEDAS data showed that 38 patients (42.75%) had a TIMI 2-3 at baseline and 51 patients (57.3%) a TIMI 0-1. This differs to the DIAS-2 data where 70.4% of patients had a baseline TIMI 2-3. The highest percentage of baseline TIMI 2-3 was found in the 90 µg/kg (74.1%) and and the lowest in the 125 µg/kg group (64.5%) (see **table 1**).

Although desmoteplase was not better than placebo in the overall DIAS-2 subjects population, exploring the subgroup of subjects with proximal artery occlusion of TIMI of 0 or 1 or high grade stenosis at baseline revealed an improved response for desmoteplase over placebo (placebo: 17.6%, 90 µg/kg: 35.7%, 125 µg/kg: 27.3%). In the pooled population of DIAS/DEDAS/DIAS-2, desmoteplase showed a dose-dependent effect over placebo for TIMI 0-1 and TIMI 2 but not for TIMI 3.

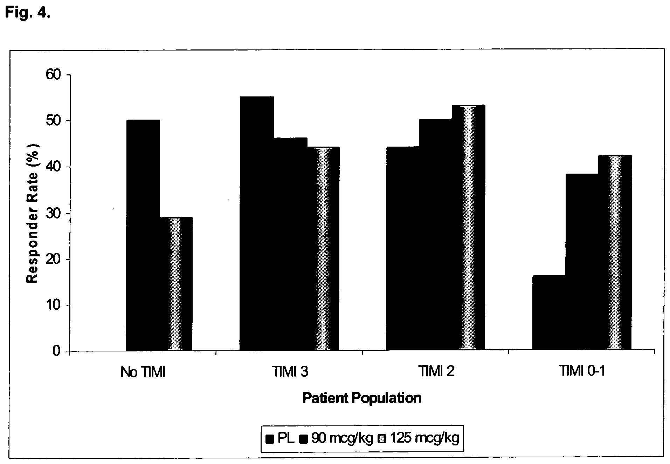

These data are shown in **tables 2a to** c and in **fig. 4****.**

### Responder rates vs. mismatch volume

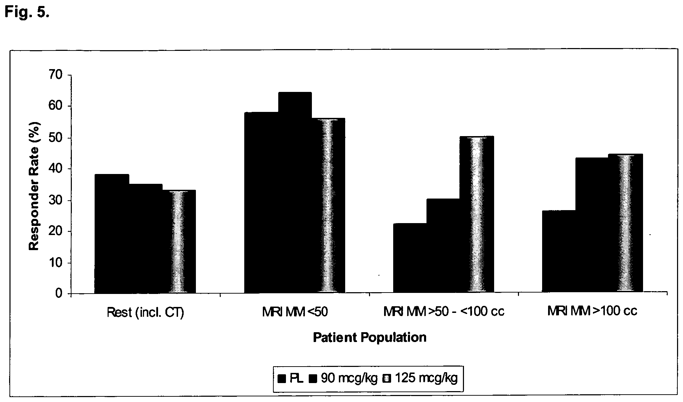

As already mentioned above the absolute mismatch volume in DIAS-2 was inversely related to the placebo response rate, so that patients with a smaller mismatch volume (i.e. 50 cc or less) showed a higher placebo response rate. In accordance, a subgroup analysis including the MRI analysed patients of DIAS/DEDAS/DIAS-2 shows that a dose-dependent response of desmoteplase over placebo can be observed for patients with absolute mismatch volumes between 50 cc and 100 cc and for patients with a mismatch volume greater than 100 cc, whereas desmoteplase was not significantly better than placebo in the subgroup with less than 50 cc absolute mismatch volume (**fig. 5**).

### Correlation between TIMI grade and mismatch volume /NIHSS

In the DIAS-2 study the patients subgroup with TIMI 0-1 exhibited a base line NIHSS of 13.0, whereas the patients with TIMI 2-3 showed a base line NIHSS of 9.0 (**table 3**). This correlation is based on the fact that patients with a more severe occlusion are more likely to have a more severe infarct. Accordingly the TIMI grade shows also a correlation to the absolute mismatch volume, since patients with TIMI 0-1 exhibit a mismatch volume of 167.7 cc and patients with TIMI 2-3 a mismatch volume of 53.5 cc. (**table 3**).

### TIMI grade and mismatch-related protocol violations

The analysis of the site maps revealed that in the DIAS-2 study 23 patients exhibited no mismatch/apparent penumbra. 11 of 23 patients of the patients without penumbra were responders.

Conclusions: These data show that patients with obvious occlusions on TIMI (0-1) have a lower chance to recover without thrombolytic therapy whereas those with baseline TIMI 2-3 are likely to achieve good recovery even without therapeutic intervention. Based on the re-analysis of the TIMI-grades at baseline that correlate with the NIHSS score and the absolute mismatch volume, the TIMI grades constitute an important factor influencing the outcome of DIAS-2.

**TABLE LEGENDS**

| | |
|---|---|
| Table 1. | Baseline characteristics for the DIAS-2 study. |
| Table 2a. | Responder rate per TIMI group in the DIAS-2 study in comparison to to the pooled patient poluation of the DIAS and DEDAS study. |
| Table 2b. | Responder rate per TIMI group in the DIAS-2 study. |
| Table 2c. | Responder rate per TIMI group (only M1 readings) in the DIAS-2 study in comparison to to the pooled patient poluation of the DIAS and DEDAS study. |
| Table 3. | TIMI grade vs. NIHSS and mismatch volume in the DIAS-2 study. |
| Table. 4: | Overview DIAS/DEDAS & DIAS-2 shows that DIAS-2 includes milder stroke with smaller mismatch volumes and absence of vessel occlusion. |

### FIGURE LEGENDS

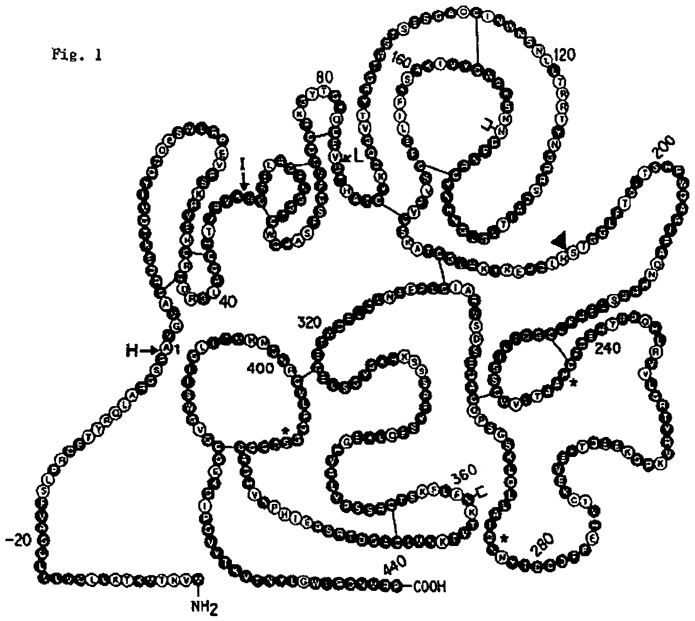

- Fig. 1:: Structure of the DSPA alpha1 protein.

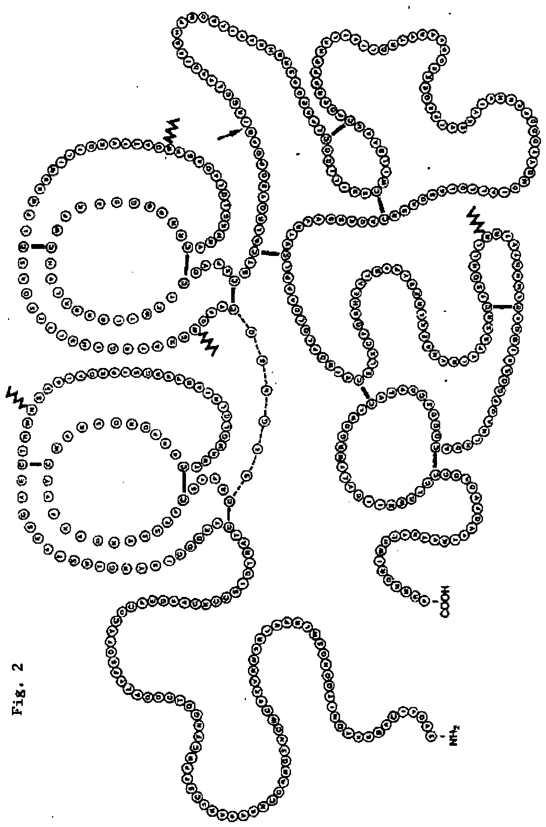

- Fig. 2:: Structure of the alteplase protein.
- Fig. 3:: Amino acid sequence of the mature DSPA alpha1 protein.
- Fig. 4:: Responder rate according to TIMI in the poooled patient population of the DIAS, DEDAS and DIAS-2 study.
- Fig. 5:: Responder rate according to MRI mismatch in the poooled patient population of the DIAS, DEDAS and DIAS-2 study.

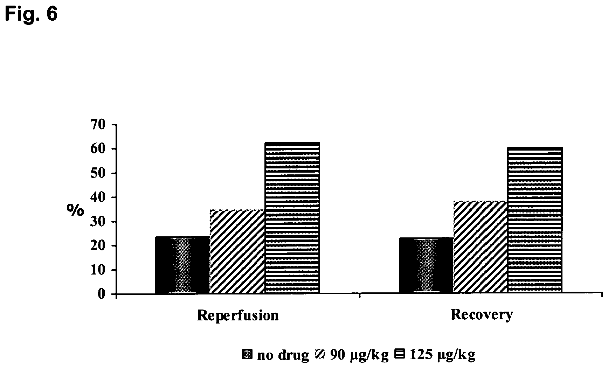

- Fig. 6:: DIAS/DEDAS: main results.

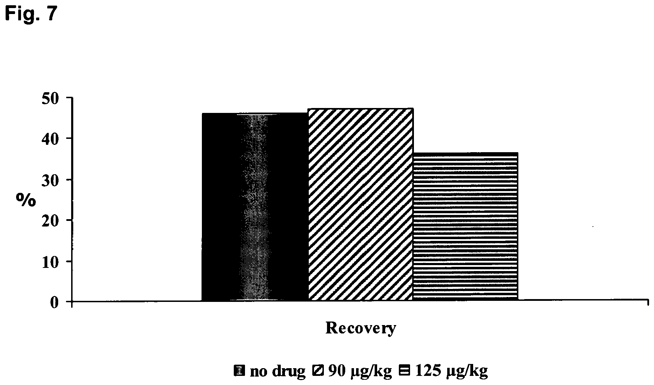

- Fig. 7:: DIAS-2: main results.

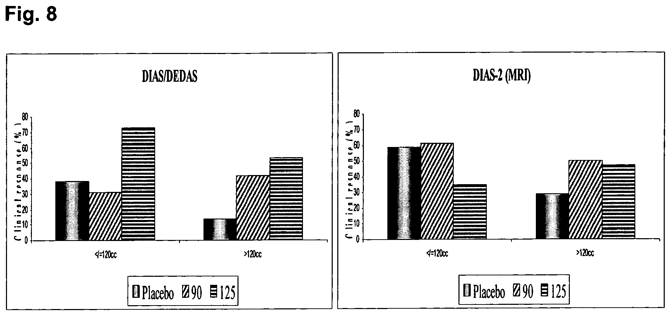

- Fig. 8:: Clinical Response by Mismatch Volume (≤ 120 cc versus >120 cc): At high mismatch volumes, both Desmoteplase doses are associated with significant improvement over placebo.

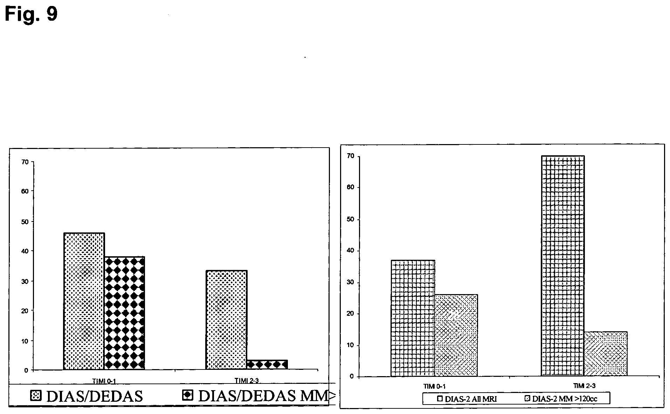

- Fig. 9:: TIMI Distribution in patients with Mismatch Volume >120 cc At high mismatch volumes, the majority of patients with no vessel occlusion/lowgrade stenosis are excluded.

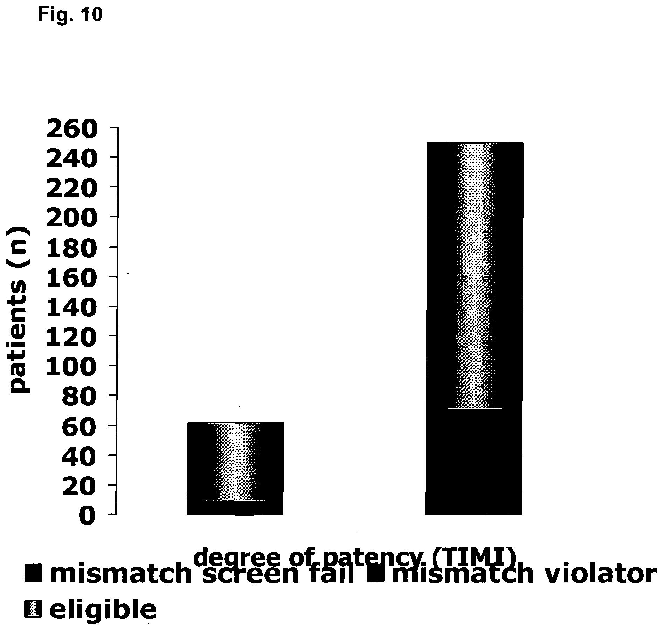

- Fig. 10:: Mismatch versus Vessel Occlusion: DIAS-2 Selecting patients with TIMI 0-1 = Vast majority with mismatch.

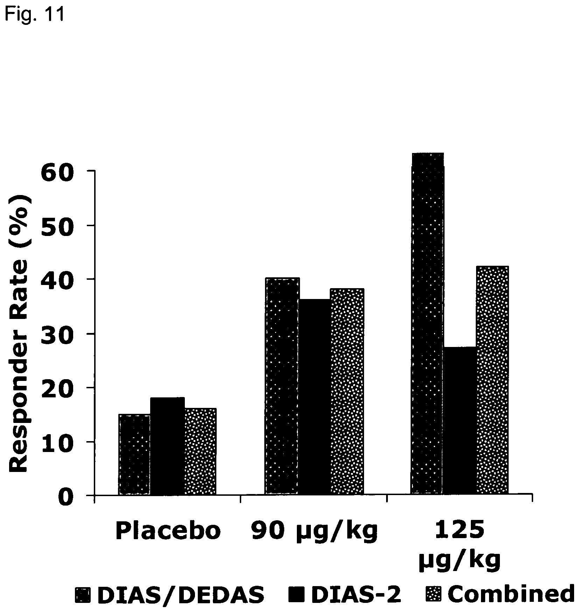

- Fig. 11:: Clinical response in patients with vessel occlusion/high-grade stenosis (TIMI 0-1). In TIMI 0-1 population, clinical response for desmoteplase 90 µg/kg and placebo was consistent effect across all studies. Overall effect size for 90 µg/kg : 22%.

**Table 1**

| | Placebo | 90µg/kg | 125µg/kg | Overall |
|---|---|---|---|---|
| NIHSS (median) | 9.0 | 9.0 | 9.0 | 9.0 |
| Age (median) [yrs] | 73.0 | 71.0 | 73.5 | - |
| Male [%] | 58.7 | 47.4 | 43.9 | 50.0 |
| Female [%] | 41.3 | 52.6 | 56.1 | 50.0 |
| BL TIMI 0-1 [%] | 27.0 | 25.9 | 35.5 | 29.6 |
| BL TIMI 2-3 [%] | 73.0 | 74.1 | 64.5 | 70.4 |

**Table 2a**

| | | TIMI 0-1 | | | | TIMI 2-3 | | | |
|---|---|---|---|---|---|---|---|---|---|
| | | n | BL NIHSS (median) | Responder Rate | | n | BL NIHSS (median) | Responder Rate | |
| | | | | n | % | | | n | % |
| | Placebo | 20 | 14.0 | 3 | 15.0 | 14 | 8.0 | 5 | 35.7 |
| | 90 µg/kg | 15 | 14.0 | 6 | 40.0 | 13 | 9.0 | 5 | 38.5 |
| | 125 µg/kg | 16 | 13.5 | 10 | 62.5 | 11 | 8.0 | 8 | 72.7 |
| | ∑ DSPA | 31 | 14.0 | 16 | 51.6 | 24 | 8.5 | 13 | 54.2 |
| | Total | 51 | 14.0 | 19 | 37.3 | 38 | 8.0 | 18 | 47.4 |
| | Placebo | 17² | 14.0 | 3 | 17.6 | 46² | 8.0 | 26 | 56.5 |
| | 90 µg/kg | 14² | 14.0 | 5 | 35.7 | 40¹ | 9.0 | 20 | 50.0 |
| | 125 µg/kg | 22⁴ | 9.5 | 6 | 27.3 | 40¹⁰ | 9.0 | 16 | 40.0 |
| | ∑ DSPA | 36⁶ | 10.5 | 11 | 30.6 | 80¹¹ | 9.0 | 36 | 45.0 |
| | Total | 53⁸ | 13.0 | 14 | 26.4 | 126¹³ | 9.0 | 62 | 49.2 |

**Table 2b**

| DIAS-2 | TIMI 0-1 | | | | TIMI 2 | | | | TIMI 3 | | | |
|---|---|---|---|---|---|---|---|---|---|---|---|---|
| | n | BL NIHSS (median) | Responder Rate | | n | BL NIHSS (median) | Response Rate | | n | BL NIHSS (median) | Responder Rate | |
| | | | n | % | | | n | % | | | n | % |
| Placebo | 17² | 14.0 | 3 | 17.6 | 16¹ | 8.5 | 8 | 50.0 | 30¹ | 8.0 | 18 | 60.0 |
| 90 µg/kg | 14² | 14.0 | 5 | 35.7 | 13 | 10.0 | 6 | 46.2 | 27¹ | 8.0 | 14 | 51.9 |
| 125 µg/kg | 22⁴ | 9.5 | 6 | 27.3 | 15³ | 11.0 | 7 | 46.7 | 25⁶ | 8.0 | 9 | 36.0 |
| ∑ DSPA | 36⁶ | 10.5 | 11 | 30.6 | 28³ | 11.0 | 13 | 46.4 | 52⁷ | 8.0 | 23 | 44.2 |
| Total | 53⁸ | 13.0 | 14 | 26.4 | 44⁴ | 10.0 | 21 | 47.7 | 82⁸ | 8.0 | 41 | 50.0 |

**Table 2c**

| | | TIMI 0-1 | | | | TIMI 2-3 | | | |
|---|---|---|---|---|---|---|---|---|---|
| | | n | BL NIHSS (median) | Responder Rate | | n | BL NIHSS (median) | Responder Rate | |
| | | | | n | % | | | n | % |
| | Placebo | 16 | 15.0 | 2 | 12.5 | 13 | 8.0 | 5 | 38.5 |
| | 90 µg/kg | 10 | 14.5 | 3 | 30.0 | 12 | 9.0 | 4 | 33.3 |
| | 125 µg/kg | 12 | 13.5 | 7 | 58.3 | 9 | 9.0 | 7 | 77.7 |
| | ∑ DSPA | 22 | 14.5 | 10 | 45.5 | 21 | 9.0 | 11 | 54.2 |
| | Total | 38 | 14.5 | 12 | 31.6 | 34 | 8.5 | 16 | 47.0 |
| | Placebo | 15² | 14.0 | 3 | 20.0 | 40² | 9.0 | 20 | 50.0 |
| | 90 µg/kg | 11¹ | 13.0 | 5 | 45.5 | 34¹ | 9.0 | 16 | 47.0 |
| | 125 µg/kg | 17³ | 10.0 | 4 | 23.5 | 35⁸ | 9.0 | 12 | 34.2 |
| | ∑ DSPA | 28⁶ | 12.0 | 9 | 32.1 | 69¹¹ | 9.0 | 28 | 40.5 |
| | Total | 43⁸ | 13.0 | 12 | 28.0 | 109¹³ | 9.0 | 48 | 44.0 |

**Table 3**

| + | TIMI 0-1 | | TIMI 2-3 | |
|---|---|---|---|---|
| | BL NIHSS (median) | mismatch (median) [cc] | BL NIHSS (median) | mismatch (median) [cc] |
| Placebo | 14.0 | 173.7 | 8.0 | 48.8 |
| 90 µg/kg | 14.0 | 202.5 | 9.0 | 50.3 |
| 125 µg/kg | 9.5 | 119.3 | 9.0 | 66.0 |
| ∑ DSPA | 10.5 | 151.2 | 9.0 | 57.5 |
| Total | 13.0 | 167.7 | 9.0 | 53.5 |

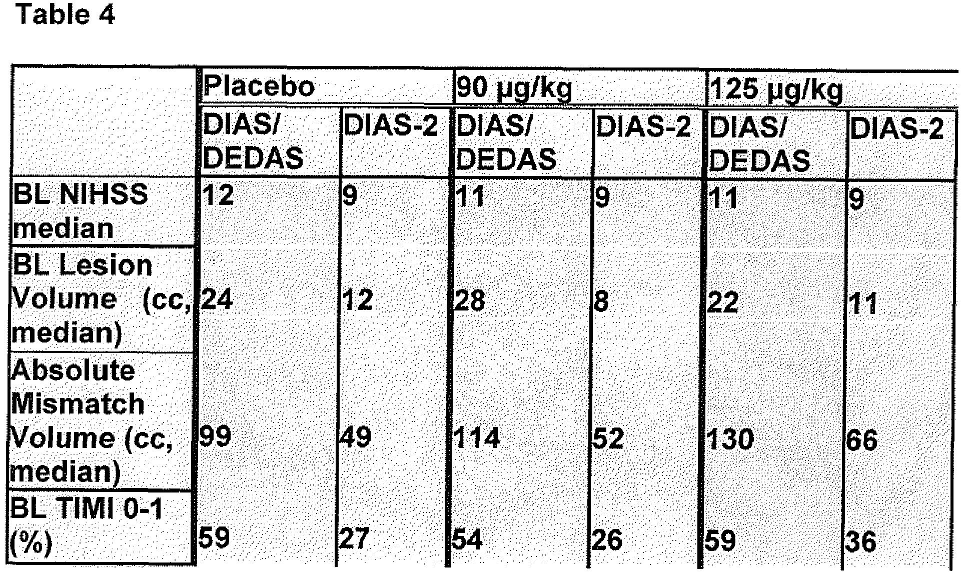

## Claims

1. Desmoteplase for use in treating stroke in a patient, whereas said patient prior to treatment is selected for exhibiting the following criteria at baseline
a. cerebral tissue at risk
b. a cerebral artery occlusion
c. a NIHSS score of at least 4, and
d. a high grade stenosis, **characterized by** a TIMI grade of 0 or 1.

2. Desmoteplase for use according to claim 1 whereas the cerebral artery occlusion or the high grade stenosis of the stroke patient is localized in the MCA, ACA or PCA or branches thereof.

3. Desmoteplase for use according to claim 2, whereas the artery occlusion or the high grade stenosis of the stroke patient is in branch M1 or M2 of the MCA, ACA or PCA.

4. Desmoteplase for use according to one of the above claims, whereas the artery occlusion of the stroke patient is of a TIMI of 0 or 1.

5. Desmoteplase for use according to one of the above claims, whereas the tissue at risk of the stroke patient is localised in the area of MCA, ACA or PCA.

6. Desmoteplase for use according to one of the above claims, whereas the patient exhibits a stroke of a NIHSS score of at least 8, preferably of between 8 and 24 (inclusive).

7. Desmoteplase for use according to one of the above claims, whereas the artery occlusion and/or the tissue at risk of the stroke patient is assessed prior treatment by individual imaging.

8. Desmoteplase for use according to one or more of the above claims, whereas the patient is further **characterized by** one or more of the following properties at baseline:
a. the acute infarction does not involve more than about 1/3 of MCA or substantially the entire ACA or PCA territory and/or
b. the absence of ICH, SAH, AV malformation, cerebral aneurysm or cerebral neoplasm,

9. Desmoteplase for use according to one or more of the above claims, whereas the plasminogen activator is administered to the patient in a dosage of about 90 to about 125 µg/kg body weight, in particular about 90 or about 125 µg/kg body weight.

10. Desmoteplase for use according to one or more of the above claims, whereby the patient is treated later than 3 hours after stroke onset or within 3 to 9 hours after the onset of stroke symptoms.

## Patentansprüche

1. Desmoteplase zur Verwendung bei der Behandlung von Schlaganfall bei einem Patienten, wobei der Patient vor der Behandlung danach ausgewählt wird, dass er bei Behandlungsbeginn die folgenden Kriterien erfüllt:
a. gefährdetes Gehirngewebe
b. Okklusion einer Zerebralarterie
c. NIHSS-Wert von mindestens 4, und
d. hochgradige Stenose, **gekennzeichnet durch** einen TIMI-Grad von 0 oder 1.

2. Desmoteplase zur Verwendung gemäß Anspruch 1, wobei die Okklusion einer Zerebralarterie oder die hochgradige Stenose des Schlaganfallpatienten in der MCA, ACA oder PCA oder Zweigen davon lokalisiert ist.

3. Desmoteplase zur Verwendung gemäß Anspruch 2, wobei die Okklusion der Arterie oder die hochgradige Stenose des Schlaganfallpatienten in Zweig M1 oder M2 der MCA, ACA oder PCA vorliegt.

4. Desmoteplase zur Verwendung gemäß einem der vorhergehenden Ansprüche, wobei die Okklusion der Arterie des Schlaganfallpatienten einen TIMI von 0 oder 1 aufweist.

5. Desmoteplase zur Verwendung gemäß einem der vorhergehenden Ansprüche, wobei das gefährdete Gewebe des Schlaganfallpatienten im Bereich der MCA, ACA oder PCA lokalisiert ist.

6. Desmoteplase zur Verwendung gemäß einem der vorhergehenden Ansprüche, wobei der Patient einen Schlaganfall mit einem NIHSS-Wert von mindestens 8 aufweist, bevorzugt zwischen 8 und 24 (inklusive).

7. Desmoteplase zur Verwendung gemäß einem der vorhergehenden Ansprüche, wobei die Okklusion der Arterie und/oder das gefährdete Gewebe des Schlaganfallpatienten vor der Behandlung durch individuelle Bildgebung beurteilt wird.

8. Desmoteplase zur Verwendung gemäß einem oder mehreren der vorhergehenden Ansprüche, wobei der Patient bei Behandlungsbeginn weiter durch eine oder mehrere Eigenschaften gekennzeichnet ist:
a. der akute Infarkt betrifft nicht mehr als ungefähr 1/3 der MCA- oder im Wesentlichen die gesamte ACA- oder PCA-Region und/oder
b. die Abwesenheit von ICH, SAH, AV Fehlbildung, zerebralem Aneurysma oder zerebralem Neoplasma.

9. Desmoteplase zur Verwendung gemäß einem oder mehreren der vorhergehenden Ansprüche, wobei der Plasminogenaktivator dem Patienten in einer Dosierung von ungefähr 90 bis ungefähr 125 µg/kg Körpergewicht, insbesondere ungefähr 90 oder ungefähr 125 µg/kg Körpergewicht verabreicht wird.

10. Desmoteplase zur Verwendung gemäß einem oder mehreren der vorhergehenden Ansprüche, wobei der Patient später als 3 Stunden nach dem Einsetzen des Schlaganfalls oder innerhalb von 3 bis 9 Stunden nach dem Einsetzen von Schlaganfallsymptomen behandelt wird.

## Revendications

1. Desmoteplase pour traiter l'AVC chez un patient, où ledit patient avant le traitement du patient est choisi pour présenter les critères suivants au départ
a. tissu cérébral à risque
b. occlusion de l'artère cérébrale
c. score NIHSS d'au moins 4, et
d. sténose de grade élevé, **caractérisée par** un degré de TIMI de 0 ou 1.

2. Desmoteplase pour utilisation selon la revendication 1, où l'occlusion de l'artère cérébrale ou la sténose de grade élevé du patient atteint d'une AVC sont localisées dans les zones MCA, ACA ou PCA ou des ramifications de ces zones.

3. Desmoteplase pour utilisation selon la revendication 2, où l'occlusion de l'artère cérébrale ou la sténose de grade élevé du patient atteint d'une AVC est située dans les ramifications M1 ou M2 des zones MCA, ACA ou PCA.

4. Desmoteplase pour utilisation selon l'une des revendications susmentionnées, où l'occlusion de l'artère du patient atteint d'une AVC présente un TIMI de 0 ou 1.

5. Desmoteplase pour utilisation selon l'une des revendications susmentionnées, où le tissu à risque du patient atteint d'une AVC est localisé dans les zones MCA, ACA ou PCA.

6. Desmoteplase pour utilisation selon l'une des revendications susmentionnées, où que le patient présente un score NIHSS d'au moins 8, de préférence compris, entre 8 et 24 (inclus).

7. Desmoteplase pour utilisation selon l'une des revendications susmentionnées, où l'occlusion artérielle et/ou le tissu à risque du patient atteint d'une AVC sont évalués avant le traitement par imagerie individuelle.

8. Desmoteplase pour utilisation selon l'une ou plusieurs des revendications susmentionnées, où le patient est encore **caractérisé par** une ou plusieurs des propriétés suivantes au départ ;
a. l'infarctus aiguë n'implique pas plus qu'environ 1/3 de MCA ou substantiellement l'ensemble du territoire ACA ou APC et/ou
b. l'absence de l'ICH, SAH, malformation AV, anévrisme cérébral ou néoplasme cérébral.

9. Desmoteplase pour utilisation selon l'une ou plusieurs des revendications susmentionnées, où l'activateur du plasminogène est administré au patient dans un dosage d'environ 90 à environ 125 µg/kg de poids corporel, en particulier d'environ 90 ou 125 µg/kg de poids corporel.

10. Desmoteplase pour utilisation selon l'une ou plusieurs des revendications susmentionnées, où le patient est traité plus tard que 3 heures après l'apparition de l'AVC ou dans les 3 à 9 heures après l'apparition des symptômes d'accident vasculaire cérébral.


### SEQUENCE LISTING

<110> PAION Deutschland GmbH
<120> Novel patient subgroups for thrombolysis
<130> 49 623 K
<150> EP 07 020 401.1
   <151> 2007-10-18
<150> EP 07 022 867.1
   <151> 2007-11-26
<150> EP 08 017 954.2
   <151> 2008-10-15
<160> 1
<170> PatentIn version 3.3
<210> 1
   <211> 441
   <212> PRT
   <213> Desmodus rotundus
<400> 1
   Ala Tyr Gly Val Ala Cys Lys Asp Glu Ile Thr Gln Met Thr Tyr Arg
   1               5                   10                  15
   Arg Gln Glu Ser Trp Leu Arg Pro Glu Val Arg Ser Lys Arg Val Glu
               20                  25                  30
   His Cys Gln Cys Asp Arg Gly Gln Ala Arg Cys His Thr Val Pro Val
           35                  40                  45
   Asn Ser Cys Ser Glu Pro Arg Cys Phe Asn Gly Gly Thr Cys Trp Gln
       50                  55                  60
   Ala Val Tyr Phe Ser Asp Phe Val Cys Gln Cys Pro Ala Gly Tyr Thr
   65                  70                  75                  80
   Gly Lys Arg Cys Glu Val Asp Thr Arg Ala Thr Cys Tyr Glu Gly Gln
                   85                  90                  95
   Gly Val Thr Tyr Arg Gly Thr Trp Ser Thr Ala Glu Ser Arg Val Glu
               100                 105                 110
   Cys Ile Asn Trp Asn Ser Ser Leu Leu Thr Arg Arg Thr Tyr Asn Gly
           115                 120                 125
   Arg Met Pro Asp Ala Phe Asn Leu Gly Leu Gly Asn His Asn Tyr Cys
       130                 135                 140
   Arg Asn Pro Asn Gly Ala Pro Lys Pro Trp Cys Tyr Val Ile Lys Ala
   145                 150                 155                 160
   Gly Lys Phe Thr Ser Glu Ser Cys Ser Val Pro Val Cys Ser Lys Ala
                   165                 170                 175
   Thr Cys Gly Leu Arg Lys Tyr Lys Glu Pro Gln Leu His Ser Thr Gly
               180                 185                 190
   Gly Leu Phe Thr Asp Ile Thr Ser His Pro Trp Gln Ala Ala Ile Phe
           195                 200                 205
   Ala Gln Asn Arg Arg Ser Ser Gly Glu Arg Phe Leu Cys Gly Gly Ile
       210                 215                 220
   Leu Ile Ser Ser Cys Trp Val Leu Thr Ala Ala His Cys Phe Gln Glu
   225                 230                 235                 240
   Ser Tyr Leu Pro Asp Gln Leu Lys Val Val Leu Gly Arg Thr Tyr Arg
                   245                 250                 255
   Val Lys Pro Gly Glu Glu Glu Gln Thr Phe Lys Val Lys Lys Tyr Ile
               260                 265                 270
   Val His Lys Glu Phe Asp Asp Asp Thr Tyr Asn Asn Asp Ile Ala Leu
           275                 280                 285
   Leu Gln Leu Lys Ser Asp Ser Pro Gln Cys Ala Gln Glu Ser Asp Ser
       290                 295                 300
   Val Arg Ala Ile Cys Leu Pro Glu Ala Asn Leu Gln Leu Pro Asp Trp
   305                 310                 315                 320
   Thr Glu Cys Glu Leu Ser Gly Tyr Gly Lys His Lys Ser Ser Ser Pro
                   325                 330                 335
   Phe Tyr Ser Glu Gln Leu Lys Glu Gly His Val Arg Leu Tyr Pro Ser
               340                 345                 350
   Ser Arg Cys Ala Pro Lys Phe Leu Phe Asn Lys Thr Val Thr Asn Asn
           355                 360                 365
   Met Leu Cys Ala Gly Asp Thr Arg Ser Gly Glu Ile Tyr Pro Asn Val
       370                 375                 380
   His Asp Ala Cys Gln Gly Asp Ser Gly Gly Pro Leu Val Cys Met Asn
   385                 390                 395                 400
   Asp Asn His Met Thr Leu Leu Gly Ile Ile Ser Trp Gly Val Gly Cys
                   405                 410                 415
   Gly Glu Lys Asp Val Pro Gly Val Tyr Thr Lys Val Thr Asn Tyr Leu
               420                 425                 430
   Gly Trp Ile Arg Asp Asn Met His Leu
           435                 440